# EUROPEAN PATENT APPLICATION

(11) **EP 2 090 662 A2**
(43) Date of publication of application: **19.08.2009**
(21) Application number: 09156643.0
(22) Date of filing: 04.04.2007
(51) Int. Cl.: C12P 13/12, C12N 15/82, C12N 15/11

(54) **Process for the production of a fine chemical**

(30) Priority: 05.04.2006 EP 06112485; 07.04.2006 EP 06112495; 12.04.2006 EP 06112737; 15.05.2006 EP 06114210; 18.05.2006 EP 06114273; 18.05.2006 EP 06114252; 18.05.2006 EP 06114258; 19.05.2006 EP 06114677; 24.05.2006 EP 06117394; 13.06.2006 EP 06115524; 14.06.2006 EP 06115416; 07.07.2006 EP 06116792; 07.07.2006 EP 06116788; 10.07.2006 EP 06116881; 20.07.2006 EP 06117578; 27.07.2006 EP 06117985; 28.07.2006 EP 06118026; 31.07.2006 EP 06118188; 02.08.2006 EP 06118329; 03.08.2006 EP 06118398; 04.08.2006 EP 06118424; 07.08.2006 EP 06118500; 09.08.2006 EP 06118634; 11.08.2006 EP 06118776; 16.08.2006 EP 06119016; 18.07.2006 EP 06119134; 21.08.2006 EP 06119211; 24.08.2006 EP 06119448; 25.08.2006 EP 06119512; 30.08.2006 EP 06119747; 07.09.2006 EP 06120256; 14.09.2006 EP 06120634; 19.09.2006 EP 06120898; 22.09.2006 EP 06121097; 26.09.2006 EP 06121250; 27.09.2006 EP 06121334; 29.09.2006 EP 06121485; 02.10.2006 EP 06121594; 02.10.2006 EP 06121602; 27.10.2006 EP 06123065; 04.01.2007 EP 07100082; 05.01.2007 EP 07100145; 11.01.2007 EP 07100403; 17.01.2007 EP 07100640; 17.01.2007 EP 07100643; 19.01.2007 EP 07100782; 22.01.2007 EP 07100934; 23.01.2007 EP 07100952; 23.01.2007 EP 07100951; 25.01.2007 EP 07101147; 29.01.2007 EP 07101328; 29.01.2007 EP 07101330; 31.01.2007 EP 07101454; 01.02.2007 EP 07101536; 02.02.2007 EP 07101615; 05.02.2007 EP 07101737; 05.02.2007 EP 07101712; 08.02.2007 EP 07101935; 08.02.2007 EP 07101936; 12.02.2007 EP 07102156; 13.02.2007 EP 07102271; 20.02.2007 EP 07102738; 21.02.2007 EP 07102812; 26.02.2007 EP 07103037; 28.03.2007 EP 07105136; 04.04.2007 EP 07857190
(62) Divisional of application: 07857190.8
(71) Applicant: Metanomics GmbH, 10589 Berlin (DE)
(72) Inventor: Puzio, Piotr, 9030 Mariakerke (BE); Blau, Astrid, 14532 Stahnsdorf (DE); Walk, Tilmann B., 14532 Kleinmachnow (DE); Gipsmans, Martijn, 14471 Potsdam (DE); Haake, Volker, 12209 Berlin (DE); Weig, Alfons, 14612 Falkensee (DE); Plesch, Gunnar, 14482 Potsdam (DE); Ebneth, Marcus, 10115 Berlin (BE)
(74) Representative: Fitzner, Uwe

(57) **Abstract**

The present invention relates to a process for the production of a fine chemical in an organism such as a microorganism, a non-human animal or plant, or in a part thereof. The invention furthermore relates to nucleic acid molecules, antisense, RNAi, snRNA, dsRNA, siRNA, miRNA, ta-siRNA, cosuppression molecule, or ribozyme molecules, or polypeptides, nucleic acid constructs, vectors, antibodies, host cells, plant tissue, propagation material, harvested material, plants, microorganisms as well as agricultural compositions and to their use.

## Description

-- the description contains several thousand pages of sequence listings and is published in electronic form only --

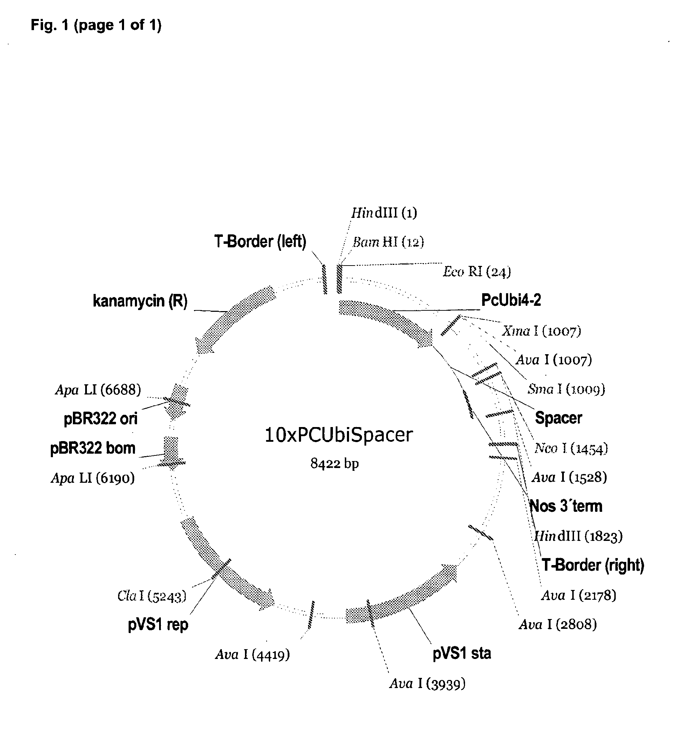

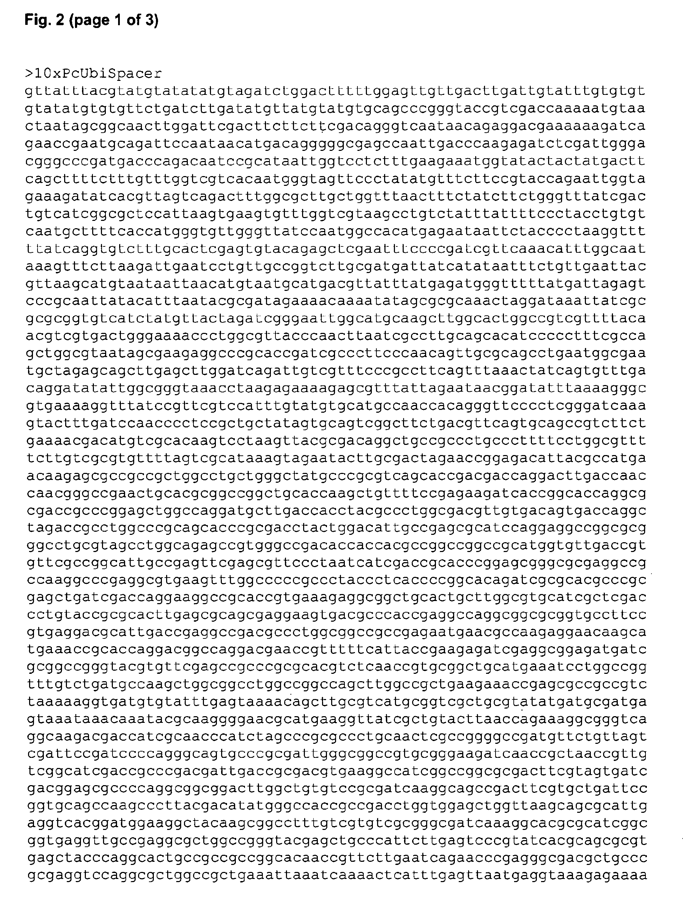

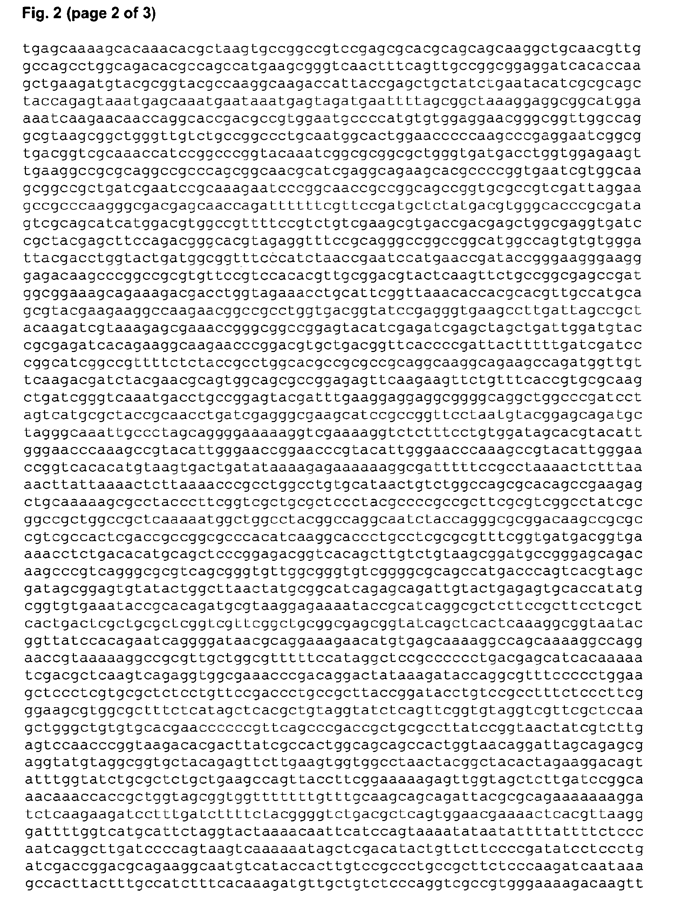

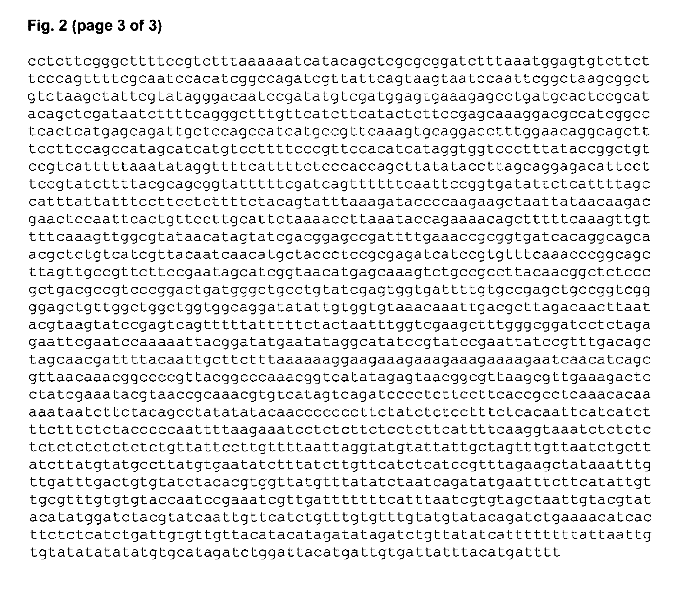

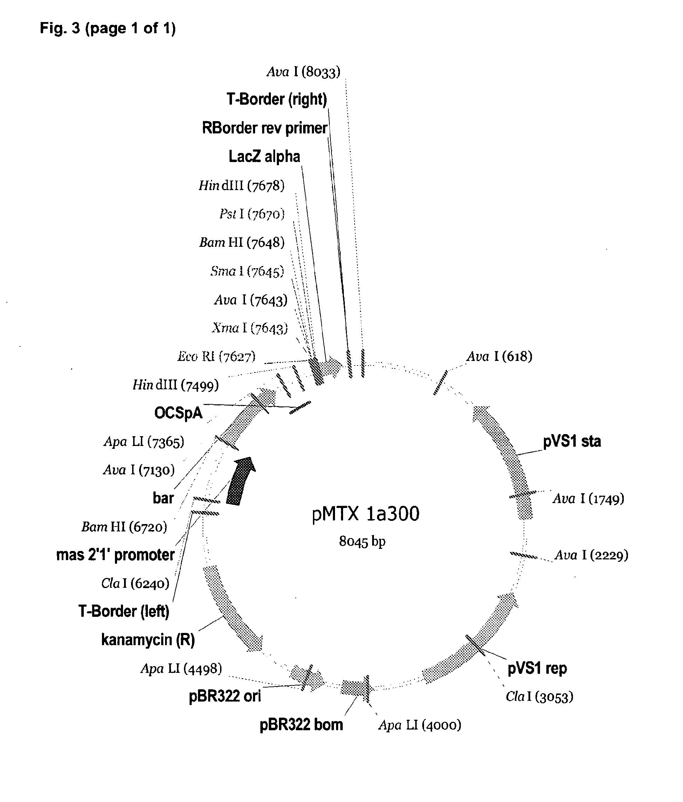

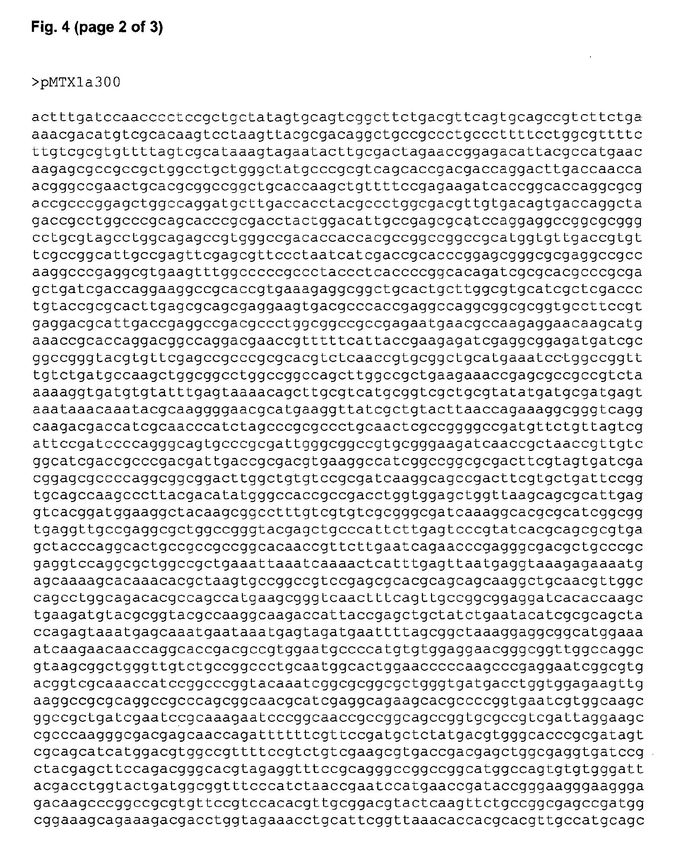

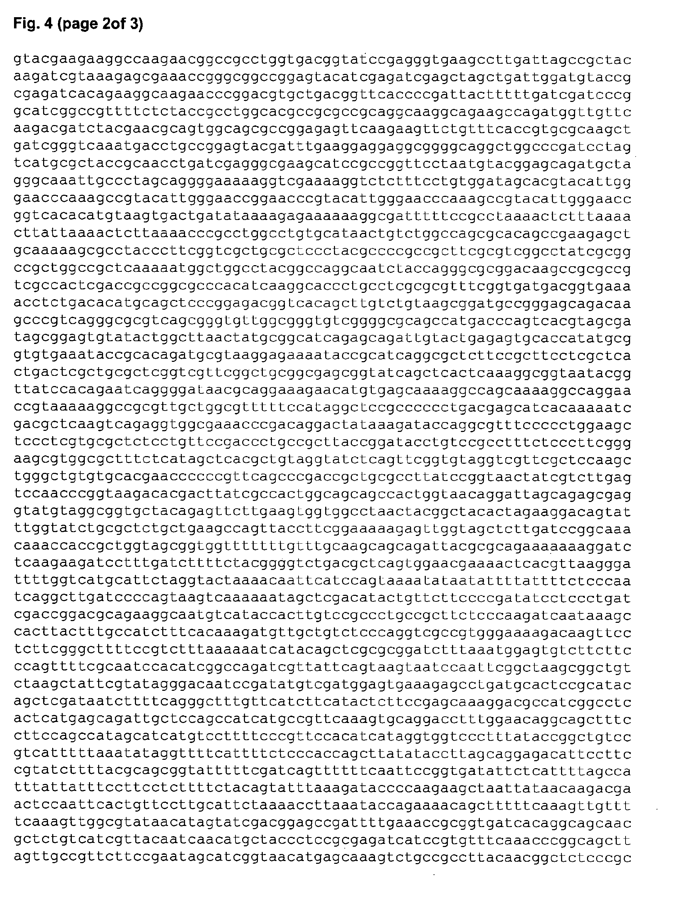

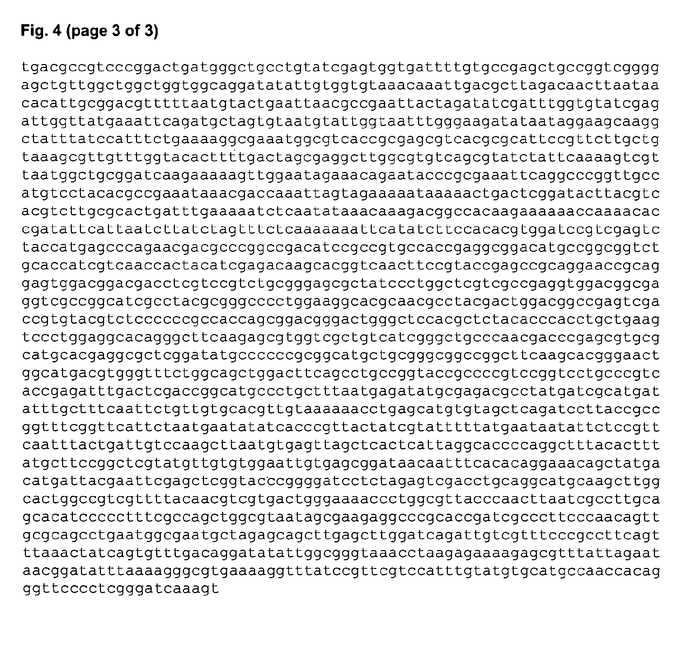

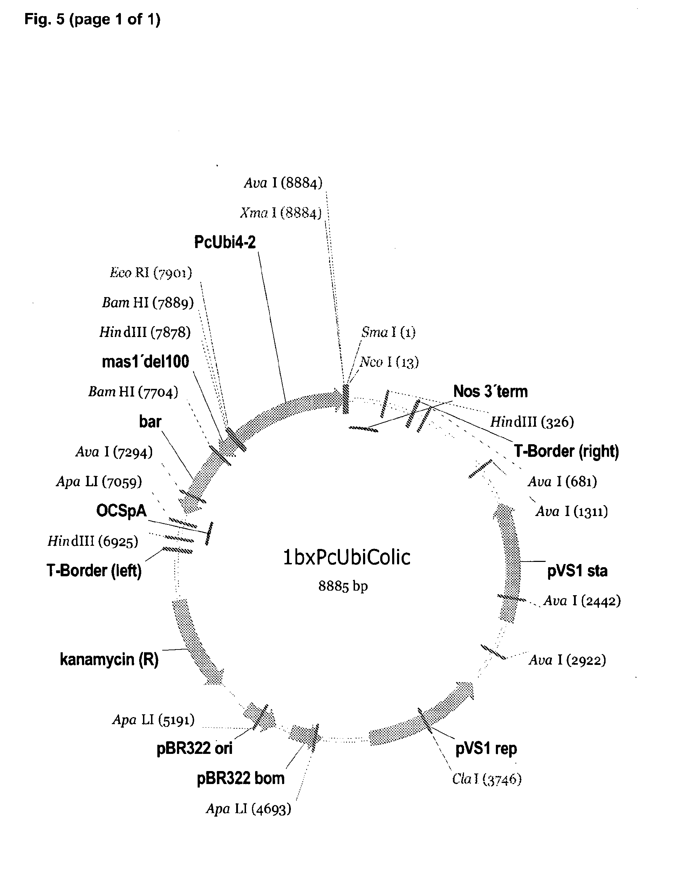

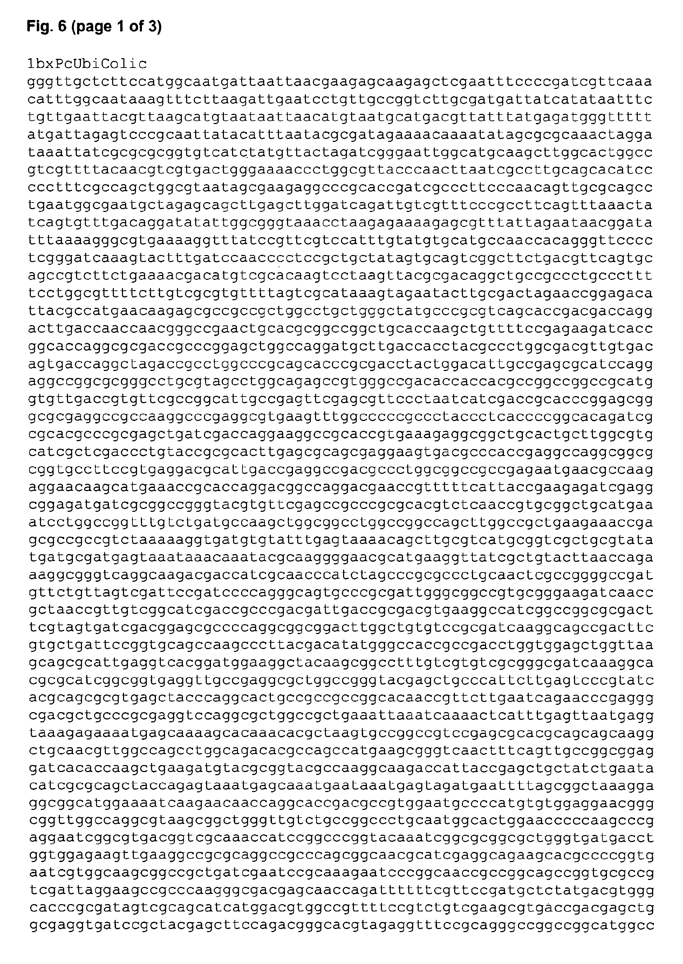

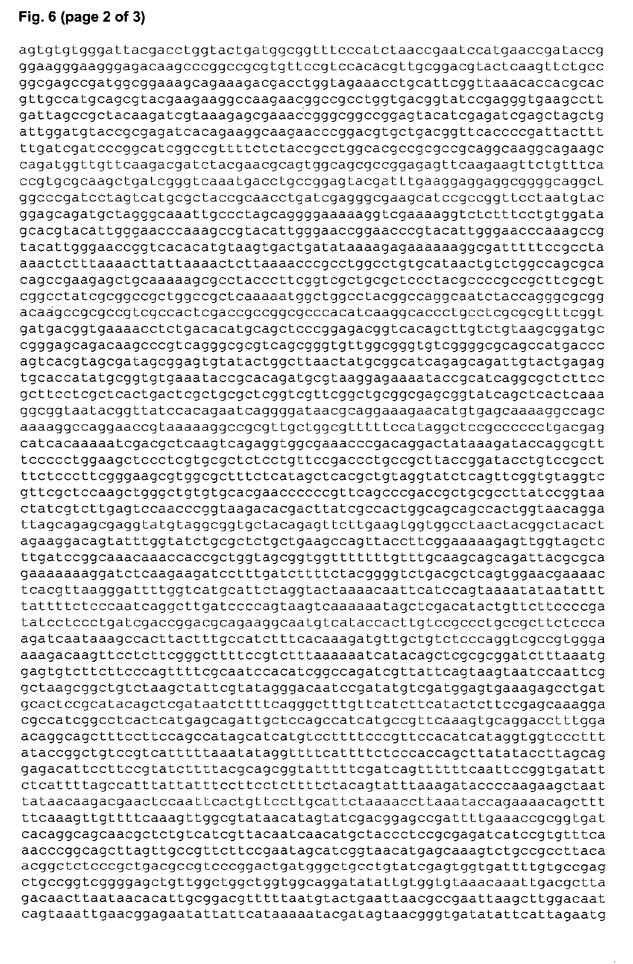

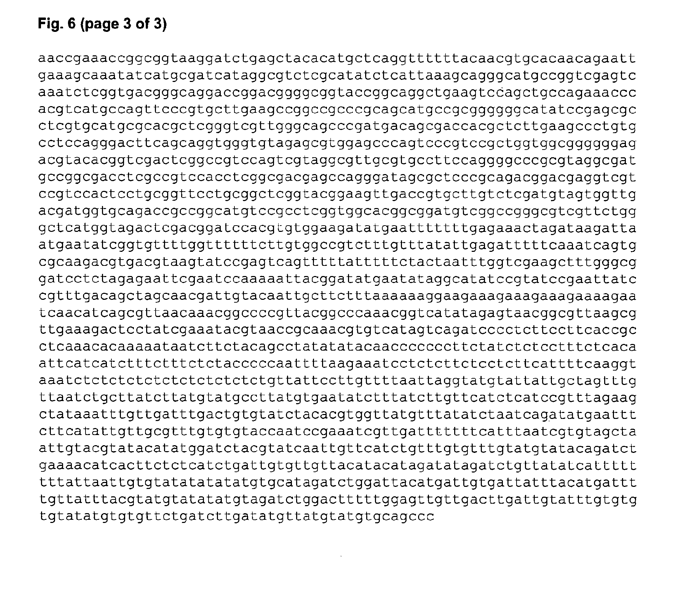

## Claims

1. A process for the production of methionine, which comprises the following steps:
a) Reducing, repressing or deleting of one or more activities selected from the group consisting of: 3-isopropylmalate dehydrogenase / oxidoreductase, 2-isopropylmalate synthase (IMS3), anion exchanger, aspartyl protease, AT1G13880-protein, At1g23780-protein, At1g27695-protein, At3g12850-protein, At3g20380-protein, At3g55990-protein, At3g59340-protein, At4g10350-protein, AT4G14713-protein, At4g16141-protein, AT4G20940-protein, At4g34770-protein, AT5G26850-protein, ATP binding protein (CPN60A), ATP-dependent peptidase/ ATPase/ nucleoside- triphosphatase/ serine-type endopeptidase, ATR2-protein, beta-galactosidase (BGAL8), calmodulin binding protein / translation elongation factor, choline kinase (ATCK1), cytidine deaminase (CDA1), DC1 domain-containing protein / protein-binding protein / zinc ion binding protein, glutamine-fructose-6-phosphate transaminase, heat shock protein binding protein / unfolded protein binding protein, kinase, L-ascorbate peroxidase (APX1), oxidoreductase, oxygen binding protein (CYP86A2), pectate lyase protein / powdery mildew susceptibility protein (PMR6), phragmoplast-associated kinesin-related protein 1 (PAKRP1), prenyltransferase (ATPPT1), presenilin family protein, ribosomal protein, transcription factor, and WRKY7 transcription factor, in a non-human organism, and
b) growing the organism under conditions which permit the production of methionine in said organism or a part thereof or in the culture medium surrounding the organism
c) and optionally the methionine synthesized by the organism is recovered or isolated

2. A process for the production of methionine, which comprises the following steps:
a) reduction, repression or deletion of the activity of
(i) a polypeptide comprising a polypeptide, a consensus sequence or at least one polypeptide motif as depicted in Application No.: 1, column 5 or 7 of Table II or of Table IV, respectively; or
(ii) an expression product of a nucleic acid molecule comprising a polynucleotide as depicted in Application No.: 1, column 5 or 7 of Table I,
(iii) or a functional equivalent of (i) or (ii);
in a non-human organism or a part thereof, and
b) growing the organism under conditions which permit the production of methionine in said organism or a part thereof or in the culture medium surrounding the organism

3. The process as claimed in any one of claims 1 to 2, comprising reducing, decreasing or deleting the expression or activity of at least one nucleic acid molecule having or encoding the activity of at least one nucleic acid molecule represented by the nucleic acid molecule as depicted in Application No.: 1, column 5 of Table I, and comprising a nucleic acid molecule which is selected from the group consisting of:
a) a nucleic acid molecule encoding the polypeptide shown in Application No.: 1, column 5 or 7 of Table II;
b) a nucleic acid molecule shown in Application No.: 1, column 5 or 7 of Table I;
c) a nucleic acid molecule, which, as a result of the degeneracy of the genetic code, can be derived from a polypeptide sequence depicted in Application No.: 1, column 5 or 7 of Table II;
d) a nucleic acid molecule having at least 30 % identity with the nucleic acid molecule sequence of a polynucleotide comprising the nucleic acid molecule shown in Application No.: 1, column 5 or 7 of Table I;
e) a nucleic acid molecule encoding a polypeptide having at least 30 % identity with the amino acid sequence of the polypeptide encoded by the nucleic acid molecule of (a) to (c) and having the activity represented by a nucleic acid molecule comprising a polynucleotide as depicted in Application No.: 1, column 5 of Table I;
f) a nucleic acid molecule encoding a polypeptide which can be isolated with the aid of monoclonal or polyclonal antibodies made against a polypeptide encoded by one of the nucleic acid molecules of (a) to (e) and having the activity represented by the nucleic acid molecule comprising a polynucleotide as depicted in Application No.: 1, column 5 of Table I;
g) a nucleic acid molecule encoding a polypeptide comprising the consensus sequence or one or more polypeptide motifs as shown in Application No.: 1, column 7 of Table IV and preferably having the activity represented by a nucleic acid molecule comprising a polynucleotide as depicted in Application No.: 1, column 5 of Table II or IV;
h) a nucleic acid molecule encoding a polypeptide having the activity represented by a protein as depicted in Application No.: 1, column 5 of Table II;
i) nucleic acid molecule which comprises a polynucleotide, which is obtained by amplifying a cDNA library or a genomic library using the primers in Application No.: 1, column 7 of Table I which do not start at their 5'-end with the nucleotides ATA and preferably having the activity represented by a nucleic acid molecule comprising a polynucleotide as depicted in Application No.: 1, column 5 of Table II or IV;
j) nucleic acid molecule encoding a polypeptide, the polypeptide being derived by substituting, deleting and/or adding one or more amino acids of the amino acid sequence of the polypeptide encoded by the nucleic acid molecules (a) to (d); and
k) a nucleic acid molecule which is obtainable by screening a suitable nucleic acid library under stringent hybridization conditions with a probe comprising a complementary sequence of a nucleic acid molecule of (a) or (b) or with a fragment thereof, having at least 15 nt, preferably 20 nt, 30 nt, 50 nt, 100 nt, 200 nt or 500 nt of a nucleic acid molecule complementary to a nucleic acid molecule sequence **characterized in** (a) to (d) and encoding a polypeptide having the activity represented by a protein comprising a polypeptide as depicted in Application No.: 1, column 5 of Table II;
or which comprises a sequence which is complementary thereto;
or reducing, repressing, decreasing or deleting of a expression product of a nucleic acid molecule comprising a nucleic acid molecule as depicted in (a) to (k), e.g. a polypeptide comprising a polypeptide as shown in Application No.: 1, column 5 or 7 of Table II.
or of a protein encoded by said nucleic acid molecule.

4. The process of any one of claims 1 to 3, comprising the reduction of the activity or expression of a polypeptide comprising a polypeptide encoded by the nucleic acid molecule **characterized in** claim 3 in an organism or part thereof.

5. The process of any one of claims 1 to 4, whereby the process comprises at least one step selected from the group consisting of:
(a) introducing of a nucleic acid molecule encoding a ribonucleic acid sequence, which is able to form a double-stranded ribonucleic acid molecule, whereby a fragment of at least 17 nt of said double-stranded ribonucleic acid molecule has a homology of at least 50 % to a nucleic acid molecule selected from the group of
(aa) a nucleic acid molecule as **characterized in** any one of claims 1 to 3;
(ab) a nucleic acid molecule as depicted in Application No. 1, column 5 or 7 of Table I or encoding a polypeptide as depicted in Application No. 1, column 5 or 7 of Table II, and
(ac) a nucleic acid molecule encoding a polypeptide having the activity of polypeptide depicted in Application No. 1, column 5 of Table II or encoding the expression product of a polynucleotide comprising a nucleic acid molecule as depicted in Application No. 1, column 5 or 7 of Table I;
(b) introducing an RNAi, snRNA, dsRNA, siRNA, miRNA, ta-siRNA, cosuppression molecule, ribozyme, or antisense nucleic acid molecule, whereby the RNAi, snRNA, dsRNA, siRNA, miRNA, ta-siRNA, cosuppression molecule, ribozyme, or antisense nucleic acid molecule comprises a fragment of at least 17 nt with a a homology of at least 50 % to a nucleic acid molecule selected from a group defined in section (a) of this claim.
(c) introducing of a ribozyme which specifically cleaves a nucleic acid molecule selected from the group defined in section (a) of this claim;
(d) introducing of the RNAi, snRNA, dsRNA, siRNA, miRNA, ta-siRNA, cosuppression molecule, ribozyme, or antisense nucleic acid molecule **characterized in** (b) and the ribozyme **characterized in** (c);
(e) introducing of a sense nucleic acid molecule conferring the expression of a nucleic acid molecule comprising a nucleic acid molecule selected from the group defined in any one of claims 2 to 3 or defined in section (ab) or (ac) of this claim or a nucleic acid molecule encoding a polypeptide having at least 50 % identity with the amino acid sequence of the polypeptide encoded by the nucleic acid molecule of claim 3 (a) to (c) and having the activity represented by a protein comprising a polypeptide depicted in Application No. 1, column 5 of Table II for inducing a co-suppression of the endogenous expression product;
(f) introducing a nucleic acid molecule conferring the expression of a dominant-negative mutant of a protein having the activity of a protein as depicted in Application No. 1, column 5 or 7 of Table II or comprising a polypeptide being encoded by a nucleic acid molecule as **characterized in** any one of claims 2 to 3;
(g) introducing a nucleic acid molecule encoding a factor, which binds to a nucleic acid molecule comprising a nucleic acid molecule selected from the group defined in any one of claims 2 to 3 or defined in section (ab) or (ac) of this claim conferring the expression of a protein having the activity of a protein encoded by a nucleic acid molecule as **characterized in** any one of claims 2 to 3;
(h) introducing a viral nucleic acid molecule conferring the decline of a RNA molecule comprising a nucleic acid molecule selected from the group defined in any one of claims 2 to 3 or defined in section (ab) or (ac) of this claim conferring the expression of a protein encoded by a nucleic acid molecule as **characterized in** any one of claims 2 to 3;
(i) introducing a nucleic acid construct capable to recombine with and silence, inactivate, repress or reduces the activity of an endogenous gene comprising a nucleic acid molecule selected from the group defined in any one of claims 2 to 3 or defined in section (ab) or (ac) of this claim conferring the expression of a protein encoded by a nucleic acid molecule as **characterized in** any one of claims 2 to 3;
(j) introducing a non-silent mutation in a endogenous gene comprising a nucleic acid molecule selected from the group defined in any one of claims 2 to 3 or defined in section (ab) or (ac) of this claim ; and
(k) introducing an expression construct conferring the expression of nucleic acid molecule **characterized in** any one of (a) to (i).

6. The process as claimed in any of the claims 1 to 5, wherein the organism is a transgenic organism selected from the group consisting of
- microorganisms,
- non-human animals and
- plants.

7. The process of any one of claims 1 to 6 comprising the step, introduction of a RNAi, snRNA, dsRNA, siRNA, miRNA, ta-siRNA, cosuppression molecule, ribozyme, antibody and/or antisense nucleic that has been designed to target the expression product of a gene comprising the nucleic acid molecule as **characterized in** any one of claims 2 to 3 to induce a breakdown of the mRNA of the said gene of interest and thereby silence the gene expression, or of an expression cassette ensuring the expression of the former.

8. An isolated nucleic acid molecule which comprises a nucleic acid molecule selected from the group consisting of:
a. a nucleic acid molecule which encodes a polypeptide comprising the polypeptide shown in Application No. 1, column 5 or 7 of Table I B or;
b. a nucleic acid molecule which comprising a polynucleotide shown in Application No. 1, column 5 or 7 of Table I B or;
c. a nucleic acid molecule comprising a nucleic acid sequence, which, as a result of the degeneracy of the genetic code, can be derived from a polypeptide sequence depicted in Application No. 1, column 5 or 7 of Table II B and having the activity represented by the protein depicted in Application No. 1, column 5 of Table II;
d. a nucleic acid molecule encoding a polypeptide having at least 50 % identity with the amino acid sequence of a polypeptide encoded by the nucleic acid molecule of (a) or (c) and having the activity represented by the protein depicted in Application No. 1, column 5 of Table II ;
e. a nucleic acid molecule encoding a polypeptide, which is isolated with the aid of monoclonal antibodies against a polypeptide encoded by one of the nucleic acid molecules of (a) to (c) and having the activity represented by the protein depicted in Application No. 1, column 5 of Table II;
f. a nucleic acid molecule encoding a polypeptide comprising the consensus sequence or a polypeptide motif shown in Application No. 1, column 7 of Table IV and having the biological activity represented by the protein depicted in Application No. 1, column 5 of Table II;
g. a nucleic acid molecule encoding a polypeptide having the activity represented by a protein as depicted in Application No. 1, column 5 of Table II;
h. nucleic acid molecule which comprises a polynucleotide, which is obtained by amplifying a cDNA library or a genomic library using the primers in Application No.: 1, column 7 of Table III which do not start at their 5'-end with the nucleotides ATA; and
i. a nucleic acid molecule which is obtainable by screening a suitable library under stringent hybridization conditions with a probe comprising one of the sequences of the nucleic acid molecule of (a) to (c) or with a fragment of at least 17 nt of the nucleic acid molecule **characterized in** any one of (a) to (h) and encoding a polypeptide having the activity represented by the protein depicted in Application No. 1, column 5 of Table II;
or which comprises a sequence which is complementary thereto;
whereby the nucleic acid molecule according to (a) to (i) is at least in one or more nucleotides different from the sequence depicted in Application No. 1, column 5 or 7 of Table I A and preferably which encodes a protein which differs at least in one or more amino acids from the protein sequences depicted in Application No. 1, column 5 or 7 of Table II A.

9. A RNAi, snRNA, dsRNA, siRNA, miRNA, ta-siRNA, cosuppression molecule, ribozyme or antisense nucleic acid molecule or an antibody for the reduction of the activity **characterized in** claim 1 or of the activity or expression of a nucleic acid molecule as **characterized in** any one of claims 2 to 8 or a polypeptide encoded by said nucleic acid molecule.

10. A nucleic acid construct comprising an isolated nucleic acid molecule as claimed in claim 8 or the RNAi, snRNA, dsRNA, siRNA, miRNA, ta-siRNA, cosuppression molecule, ribozyme, or antisense nucleic acid molecule of claim 9, , wherein the nucleic acid molecule is functionally linked to one or more regulatory signals.

11. A vector comprising the nucleic acid molecule claimed in claim 8 or the RNAi, snRNA, dsRNA, siRNA, miRNA, ta-siRNA, cosuppression molecule, ribozyme, or antisense nucleic acid molecule of claim 9, or the nucleic acid construct as claimed in claim 10.

12. A transgenic host cell which has been transformed stably or transiently with the vector as claimed in claim 11, or the nucleic acid molecule as claimed in claim 8 or the nucleic acid construct as claimed in claim 10.

13. A host cell or a non-human organism wherein the activity of a protein comprising a polypeptide, a consensus sequence or a polypeptide motif as depicted in Application No. 1, column 5 or 7 of Table II, preferably Table II B, or IV or a nucleic acid molecule comprising a nucleic acid molecule as depicted in Application No. 1, column 5 or 7 of Table I, preferably Table I B, is reduced.

14. An isolated polypeptide encoded by a nucleic acid molecule as claimed in claim 8 or comprising the polypeptide as depicted in Application No. 1, column 7 of Table II B.

15. Food or feed composition comprising the protein according to claim 14, the nucleic acid molecule of claim 8, the nucleic acid construct of claim 10, the vector of claim 11, the host cell of claim 13, the nucleic acid molecule **characterized in** any one of claims 2 to 3, the RNAi, snRNA, dsRNA, siRNA, miRNA, ta-siRNA, cosuppression molecule, ribozyme, or antisense nucleic acid molecule of claim 9.
